(19) Europäisches Patentamt
European Patent Office
Office européen des brevets

(11) EP 1 857 535 B1

(12) EUROPEAN PATENT SPECIFICATION

(45) Date of publication and mention of the grant of the patent:
**22.12.2010 Bulletin 2010/51**

(21) Application number: **07251650.3**

(22) Date of filing: **19.04.2007**

(51) Int Cl.:
*C11D 1/62* (2006.01) *C11D 1/88* (2006.01)
*C11D 1/94* (2006.01) *C11D 3/00* (2006.01)
*A61K 8/34* (2006.01) *A61K 8/41* (2006.01)
*A61K 8/45* (2006.01) *A61Q 19/00* (2006.01)

# (54) Antimicrobial hand wash formulations

Antimikrobielle Handwaschformulierungen

Formulations antimicrobiennes pour le lavage des mains

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **16.05.2006 US 435070**

(43) Date of publication of application:
**21.11.2007 Bulletin 2007/47**

(73) Proprietor: **Gojo Industries, Inc.**
**Akron, OH 44311 (US)**

(72) Inventors:
- **Barnhart, Ronald A.**
  **Clinton**
  **Ohio 44216 (US)**
- **Lerner, David P.**
  **Akron**
  **Ohio 44333 (US)**

(74) Representative: **Makovski, Priscilla Mary**
**Barker Brettell LLP**
**100 Hagley Road**
**Edgbaston**
**Birmingham**
**B16 8QQ (GB)**

(56) References cited:
**WO-A-00/49127** **WO-A-01/41567**
**WO-A-01/41727** **WO-A-93/00089**
**WO-A-98/30673** **WO-A-98/55092**
**WO-A-03/006071** **WO-A-03/091375**
**US-A- 5 929 024** **US-A- 6 136 304**
**US-A1- 2004 138 084**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 857 535 B1

## Description

### TECHNICAL FIELD OF THE INVENTION

The present invention resides in the art of antimicrobial hand wash formulations. More particularly, the present invention relates to a highly efficacious antimicrobial hand wash containing relatively low levels of skin-irritating surfactants and active ingredients.

### BACKGROUND OF THE INVENTION

Most antimicrobial hand wash formulations exhibiting broad spectrum activity contain surfactants, active ingredients, or both. Surfactants are employed, in part, to help solubilize the active ingredients, and to make them useful in the formulation. The surfactants are typically selected from anionic, non-ionic, amphoteric, quaternary ammonium, and amine oxide surfactants. As is generally appreciated, all of these classes of surfactants have their positive and negative properties. For example, quaternary ammonium compounds are compatible with phenol-based active ingredients such as 2,3,4'-trichloro-2'-hydroxydiphenylether (triclosan), 2,2'-dihydroxy-5,5'-dibromodiphenyl ether, p-chloro-m-xylenol (pcmx), and ortho-phenylphenol, but, when they are used, the hand washes do not foam to a great extent. Amphoterics and amine oxides are expensive. Anionics and non-ionics also tend not to interact well with the active ingredients.

Active ingredients are typically selected from bisguanidines, quaternary ammonium compounds, benzyl alcohols, trihalocarbanilides, iodine-containing compounds, and phenol-based compounds. All of these types of active ingredients have found niches in today's cosmetic markets. The phenol-based actives, such as triclosan, 2,2'-dihydroxy-5,5'-dibromodiphenyl ether, pcmx, and ortho-phenylphenol are extensively used in hand wash formulations. But, despite their extensive use, there are numerous negative associations with using these as active agents. Owing to the non-polar character of these compounds, they are sparingly soluble in an aqueous environment. Solvents, such as propylene glycol, or hydrotropes, such as sodium xylene sulfonate, are often required to incorporate them into an aqueous hand wash system. The use of solvents and hydrotropes usually cause detrimental effects to the final hand wash either via an increased cost or increased irritancy. The aqueous environment might be heated in order to increase solubility and avoid the negative effects of the solvents and hydrotropes, but adding heat is undesirable because large amounts of energy and extended manufacturing times are required. A hand wash that is low in solids would offer the advantages of reduced cost combined with a probable reduction in irritation through minimizing the use of irritating surfactants.

A second deterrent for using the phenol-based actives results from incompatibility between these compounds and commonly used surfactants, specifically anionic, non-ionic and amphoteric surfactants. Because of this adverse effect, the surfactant choices are limited to those classes remaining, namely quaternary ammonium compounds and amine oxides. These two surfactant types, however, have their detrimental properties. Quaternary ammonium compounds do not produce an aesthetically pleasing hand wash due to their inability to foam, and these compounds are comparatively much more expensive than the commonly used surfactant classes. Amine oxides, much like quaternary ammonium compounds, lack the full gamut of necessary aesthetic properties to produce a commercially viable hand wash. Although amine oxides have relatively standard flash foaming properties, the foam is not stable, and again this surfactant class is expensive compared to the commonly used surfactants. Therefore, there is a need in the art for a surfactant combination that allows for an aesthetically pleasing hand wash that also has the necessary antimicrobial properties.

Current consumer trends drive for a hand wash which copiously foams, has the desired antimicrobial properties, and does not irritate skin. To produce a hand wash that foams to the extent desired, anionic surfactants are usually added. But these surfactants usually deactivate the active ingredient and are detrimental to the skin. The next best choice for foaming enhancement are the amphoteric surfactants. Although they do not foam as greatly as the anionic surfactants, they do have better skin compatibility. Again, amphoteric surfactants are not usually compatible with the active agents. Non-ionics, although excellent in militating against irritation, do not foam to an appreciable extent, and, because of this, their use is limited. Moreover, non-ionic surfactants usually deactivate the active ingredients. Therefore, there is a need in the art for a surfactant combination which meets the desired foaming properties, is of low irritancy, and provides highly efficacious properties.

One step used to reduce irritancy in a hand wash is reducing the amount of active ingredient within the hand wash. This reduction, although reducing the irritancy of the hand wash, causes a detrimental effect on the washing properties. This reduction requires the use of other ingredients within the hand wash that have nearly no negative affect on the efficacious properties of the active agent. This minimization of negative interactions requires two possible arrangements. One, the use of other ingredients which only positively affect or at least have no negative affect on the active ingredient, or two, any ingredients which negatively impact the active ingredient are minimized.

International Patent Publication No: WO 01/41567 relates to an antimicrobial composition particularly for use as a surgical hardwash composition. The composition aims to provide a long lasting and instant antimicrobial activity

and comprises a blend of antimicrobial agents and particular surfactants and including anionic surfactants. In particular the compositions contain cationic quaternary ammonium compounds, a surfactant system of non-ionic, cationic, and optionally amphoteric surfactants and desirably a biguanide compound.

## SUMMARY OF THE INVENTION

Disclosed but not forming part of the claimed invention is an antimicrobial hand wash comprising a phenol-based active ingredient and an amphoteric surfactant selected from the group consisting of derivative compounds of a fatty amine with a nitrogen atom bound to at least one, but not greater than two, propionic acids. Specific, non-limiting examples of such acids include disodium capryloamphodiactate, cocaminpropionic acid and cocoamphodipropionic acid. Specific non-limiting examples of useful active ingredients include triclosan, 2,2'-dihydroxy-5,5'-dibromodiphenyl ether, pcmx, ortho-phenylphenol, and mixtures thereof.

Also disclosed but not forming part of the claimed invention is an antimicrobial hand wash comprising a phenol-based active ingredient and a quaternary ammonium compound selected from the group consisting of polyethylene glycol (PEG) derivatives of a quaternary ammonium salt of a fatty amine, polypropylene glycol (PPG) derivatives of a quaternary ammonium salt of a fatty amine, and mixture thereof. The PEG and PPG derivative compounds are cationic surfactants that have the uncanny ability to solubilize the active ingredient. Even upon dilution of the formulation into water, the active ingredients remain in solution. Thus, by employing the cationic PEG and/or PPG derivative surfactant, the total solids amount in the formula is minimized, which ultimately relates to reduced skin irritation. The reduction in solids content is a result of not having to employ a significant amount of additional solubilizing surfactants and/or glycols.

This invention provides an aqueous antimicrobial hand wash comprising a phenol-based active ingredient; an amphoteric surfactant ; and a quaternary ammonium compound as defined in claim 1.

In particular embodiments of the foregoing hand washes, additional compounds are employed, as generally known, to hone the aesthetic properties of the hand wash. These compounds include dyes, fragrances, foam modifying agents skin conditioning agents, including but not limited to humectants, emollients, and anti-static agents.

In accordance with this invention, a method for producing an aqueous antimicrobial hand wash as defined in claim 6 is provided. In this production process, it is not necessary to heat the premix solution to dissolve the active ingredient. As a result, creating the hand wash requires less mixing time and less energy input.

## DETAILED DESCRIPTION OF PREFERRED EMBODIMENTS

Antimicrobial hand washes in accordance with this invention are aqueous hand washes including at least one active ingredient as defined in claim 1 incorporated into water in combination with at least one surfactant as defined in claim 1. Although this describes the basic make up, as is generally common in the art, to produce an acceptable end hand wash product, multiple surfactants are employed, as are skin conditioning agents, pH adjusting agents, foam modifying agents, preservatives, dyes, fragrances.

The antimicrobial hand wash contains at least one active ingredient, which is generally appreciated as a term of art for those compounds that produce acceptable time-kill antimicrobial activity to be suitable for sanitizing. More specifically, the hand wash herein has efficacious properties against both Gram-positive and Gram-negative microorganisms. For purposes of this disclosure, the terms "active ingredient" and "actives" are to cover compositions that have greater than 2 log kill reduction on both Gram-negative bacteria, specifically *Klebsiella pheumoniae,* and Gram-positive bacteria, specifically *Staphylococcus aureus.* In this invention, phenol-based active ingredients as defined in claim 1 are employed.

A particularly useful 2-hydroxydiphenyl compound has the structure:

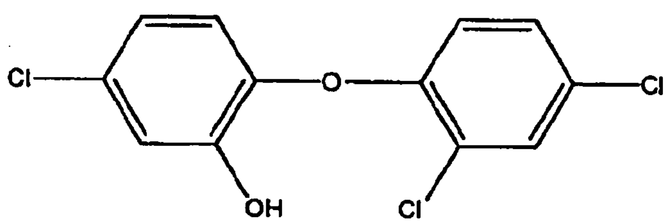

having the adopted name, triclosan, and available commercially under the tradename IRGASAN DP100, from Ciba Specialty Chemicals Corp., Greensboro, N.C. Another useful 2-hydroxydiphenyl compound is 2,2'-dihydroxy-5,5'-dibromodiphenyl ether.

Specific phenol derivatives include p-chloro-m-xylenol, and o-phenylphenol.

the phenol-based active ingredient is selected from triclosan, 2,2'-dihydroxy-5,5'-dibromodiphenyl ether, pcmx, ortho-phenylphenol, and mixtures thereof.

The hand wash formulations of this invention are typically comprised of from about .01 to 10 weight percent (wt%) of the phenol-based active ingredient. In particular embodiments, the active ingredient makes up from about .05 to 1 wt% of the formulation, and in other embodiments, from about 0.2 to 0.4 wt %.

The hand wash formulation further includes at least one amphoteric, more particularly zwitterionic, surfactant selected from the group consisting of disodium capryloamphodiactate and cocaminopropionic acid. These specific zwitterionic surfactants have unexpectedly been found to be compatible with phenol-based active ingredients according to claim 1 such that they maintain the antimicrobial efficacy of the active while still allowing for an increase in foam quality when it is desired to provide a hand wash formulation that foams. Given the substantial commercial success of foamed hand washes, it is envisioned that a foaming hand wash formulation in accordance with this invention is most preferred. The most preferred zwitterionic surfactant is cocaminopropionic acid because of its excellent compatibility with the phenol-based active ingredients.

The hand wash formulations of this invention are typically comprised of from about 0.05 to 25 weight percent (wt%) of such zwitterionic surfactants. In particular embodiments, the zwitterionic surfactant makes up from about 0.1 to 12 wt% of the formulation, and in other embodiments, from about 0.5 to 2.5 wt %.

The antimicrobial hand wash further includes a quaternary ammonium compound. More specifically, the quaternary ammonium compound is chosen due to its unique ability to at least partially dissolve the active ingredient even at low usage levels, as low as a three to one molar ratio of the quaternary compound to the phenol-based active ingredients. The quaternary ammonium compound is selected from polyethylene glycol (PEG) derivatives of a quaternary ammonium salt of a fatty amine, polypropylene glycol (PPG) derivatives of a quaternary ammonium salt of a fatty amine, and mixtures thereof. Preferably the fatty chain contains from about 6 to 20 carbon atoms. The most preferred compound is PEG-5 oleammonium methosulfate. PEG-2 oleammonium chloride and PEG-15 oleammonium chloride are also particularly preferred.

Whereas, in the prior art it has been necessary to heat the solution to solubilize the active ingredient, the disclosed quaternary ammonium compounds at least partially solubilize the active ingredient at room temperature, thus allowing the hand wash formulation to be mixed without the addition of heat. These quaternary cationic surfactants allow for a lesser amount of total surfactants than were normally required in the prior art to dissolve active ingredients. Because surfactants can be irritating to the skin, their reduction in the hand wash formulation is advantageous, providing formulations with increased efficacious properties, due to the solubilized active ingredient, and with minimal irritating ingredients, due to the use of a lesser amount of surfactants. Whereas the prior art uses a normal about of solids within a formulation being from about 12 to 30 weight percent, the preferred embodiments herein include only from about 3 to 5 weight percent solids.

The hand wash formulations of this invention are typically comprised of from about 0.01 to 10 weight percent (wt%) of the quaternary ammonium compound. In particular embodiments, the quaternary ammonium compound makes up about 0.05 to 5 wt% of the formulation, and in other embodiments, from about 0.15 to 2 wt%. The quaternary ammonium compound dissolves at least a portion of the phenol-based active ingredient, and, preferably, dissolves substantially all of the active ingredient.

An antimicrobial hand wash in accordance with this invention will include each of the ingredients disclosed above, namely, phenol-based active ingredient, zwitterionic surfactant, and quaternary ammonium compound, as disclosed. These ingredients are balance with water. However, as known, additional compounds are typically used to produce an acceptable hand wash for consumer use. These compounds include, but are not limited to, foam modifying agents, pH adjusting agents, emollients, humectants, skin conditioning agents, dyes and fragrances. Herein, they may be employed in amounts and for reasons known in the prior art. Thus, additional surfactants may be employed but are not necessary.

In embodiments disclosed but not claimed benefits are also realized by creating hand washes without all three main ingredients as just disclosed.

Although the zwitterionic surfactants do not foam inexhaustibly, they do promote foaming. Also, they have little irritation so there is no critical upper limit for the amount at which they are used in the hand wash. Although, the quaternary ammonium compound does not foam to any appreciable extent, it has unique interactions with the active ingredients (i.e., it dissolves at least a portion thereof), allowing for maximum efficacious properties.

To make hand wash formulations in accordance with prior art processes, generally all ingredients are mixed and brought to a temperature of about 60°C to 70°C, with the mixing continuing for hours to create a homogeneous mix. In accordance with this invention, a hand wash formulation can be made through a "cold" process that does not require heating formulation mixtures.

A method for producing an aqueous antimicrobial hand wash in accordance with this invention includes creating an active ingredient premix comprised of a phenol based active ingredient selected from 2,3,4-trichloro-2'-hydroxydiphenyl ether, 2,2'-dihydroxy-5,5'-dibromo diphenyl ether, p-chloro-n-xyloro orthophenylphenol, and mixtures thereof and a

quaternary ammonium compound selected from polyethylene glycol (PEG) derivatives of a quaternary ammonium salt of a fatty amine, polypropylene glycol (PPG) derivatives of a quaternary ammonium salt of a fatty amine, and mixtures thereof, wherein the quaternary ammonium compound dissolves at least a portion of the active ingredient. A second premix is made surfactant selected from containing water, disodium capryloamphodiactate and coaminopropionic acid, and any extra compounds as seen fit to create an aesthetically pleasing hand wash. These two premixes are combined and mixed until homogeneous.

The combination of the active premix and the water premix may proceed in one of two fashions, either the active premix may be added to the water premix or the water premix may be added to the active premix. Also, the combining of these two solutions may occur at any time during the addition of compounds to the water. More precisely, the two premixes may be added prior to any compound addition to the water premix, or after the addition of any number or compounds. This addition of the active premix should proceed only after the polyethylene glycol derivative dissolves at least a portion, and preferably substantially all, of the active ingredient. Through this production process, it is not necessary to heat the solution, i.e. the production of the hand wash may proceed at ambient temperature, to dissolve the active ingredient, thereby creating a hand wash which requires less mixing time. This yields cost savings via shorter turn-over time and energy savings.

**Experimental**

Example One: (outside scope of claimed invention)

The use of amphoteric surfactants in antimicrobial hand washes containing triclosan, and/or p-chloro-m-xylenol, is a rarity because these amphoteric surfactants normally deactivate the active ingredients. Unexpectedly, it has been found that there are a few amphoteric surfactants, more specifically zwitterionic surfactants, that do not inhibit the antimicrobial properties of the active ingredient. The following sample creation and testing will demonstrate this discovery.

Multiple hand wash test samples were made in accordance with the formulation provided directly below, wherein the zwitterionic surfactant was changed for each sample. The zwitterionic surfactants employed, as well as the efficacy of the resultant hand wash in reducing *Escherichia coli,* are reflected in Table 1. The process for making the samples was as follows: the active ingredient was added to the surfactant and mixed until all of the solid active ingredient dissolved into the surfactant. If required, heat was administered to dissolve the active ingredient. Water was then added to this "active premix". The samples were tested against *Escherichia coli* by inoculating a sample of the organism with a loopful of the hand wash solution for 15 seconds. A sample was then taken from the broth and incubated on a nutrient rich agar plate for 48 hours prior to counting. The formulation was as follows:

| Chemical | Amount | |
|---|---|---|
| Processed Water | q.s. to 100g | |
| Zwitterionic Surfactant | 20.00 g | |
| Triclosan | 0.30g | Ciba Specalities (Irgasan DP300) |
| Lactic Acid | q.s. to pH 5.25 | Purac (Purac HiPure USP 90%) |

Table One:

| Chemical | Company | Trade Name | Log Reduction |
|---|---|---|---|
| Disodium Capryloamphodiacetate | McIntyre Group Ltd | Mackam 2CY-75 | 5.1 |
| Cocaminopropionic acid | McIntyre Group Ltd | Mackam 151C | 2.4 |
| Coco alkyldimethyl betaine | Albright and Wilson | Empigen BB | 0.4 |
| Oleyl betaine | Chemron | Chembetaine OL | 0.3 |
| Sodium Cocoamphodiacetate | McIntyre Group Ltd | Mackam 2C-LV | 0.2 |
| Cocamidopropyl Hydroxysultaine | Chemron | Chembetaine CAS | 0.2 |
| Sodium Laurylamphoacetate | McIntyre Group Ltd | Mackam 1L | 0.1 |
| Disodium Lauramphodiacetate | McIntyre Group Ltd | Mackam 2L | 0.1 |
| Sodium Lauramphoacetate | McIntyre Group Ltd | Mackam HPL-28 | 0.1 |
| Sodium Cocoamphopropionate | McIntrye Group Ltd | Mackam CSF-CG | 0.1 |

(continued)

| Chemical | Company | Trade Name | Log Reduction |
|---|---|---|---|
| Sodium Cocoamphoacetate | McIntyre Group Ltd | Mackam 1C | 0.1 |
| Disodium Cocoamphodipropionate | McIntyre Group Ltd | Mackam 2CSF-40CG | 0.1 |
| Lauryl Hydroxysultaine | McIntyre Group Ltd | Mackam LHS | 0.1 |
| Disodium Cocoamphodiacetate | McIntyre Group Ltd | Mackam 2C | 0.0 |
| Disodium Cocoamphodipropionate | McIntyre Group Ltd | Mackam 2CSF-70 | 0.0 |
| Disodium Wheatgermamphodiacetate | McIntyre Group Ltd | Mackam 2W | 0.0 |
| Sodium Cocamphoacetate | McIntyre Group Ltd | Mackam HPC-32 | 0.0 |
| Disodium Sunfloweramphodiacetate | McIntyre Group Ltd | Mackam 2SU | 0.0 |
| Sodium Laurimindipropionate | McIntyre Group Ltd | Mackam 160C-30 | 0.0 |
| Disodium Soyamphodiacetate | McIntyre Group Ltd | Mackam 2S | 0.0 |
| Cocamidopropyl Betaine | Stepan | Amphosol HCG | 0.0 |
| Disodium Cocoamphodiacetate | McIntyre Group Ltd | Mackam 2C-LV | 0.0 |

Of the numerous zwitterionic surfactants tested only a few actually did not inhibit the active ingredient. With the diminished log reductions, the surfactants become less and less desirable for use in an antimicrobial hand wash. The class of surfactants is limited to disodium capryloamphodiacetate and those of compounds containing a nitrogen atom bound to at least one but not greater than two propionic acid groups, and a fatty acid chain from carbon length 6 to 20.

<u>Example Two</u>:

The efficacy of a particular hand wash in accordance with this invention was tested against multiple microorganisms and strains thereof. The organisms tested varied greatly, including bacterial, yeast and fungal species, and are of the type most commonly found in settings where clean, sanitized hands are most desired. The test procedure was as follows: the hand wash was diluted to 99% volume/volume of the original concentration, and this new sample was then added to a second solution containing the microorganism to be tested. This inoculation occurred at 15 and 30 seconds. The samples were then plated onto agar plates and incubated. The log reduction was calculated by comparing the values between a non-inoculated sample and the sample inoculated with the hand wash.
The hand wash contained the following ingredients:

| Chemical | Amount | |
|---|---|---|
| Processed Water | q.s. to 100g | |
| Dipropylene Glycol | 3.0 g | Huntsman (Dipropylene Glycol-LO) |
| Triclosan | 0.3 g | Ciba Specalities (Irgasan DP300) |
| PEG-5 Oleammonium Methosulfate | 0.7 g | Abitech (Accoquat OMS-5) |
| Cocaminopropionic acid | 0.2 g | McIntyre (Mackam 151C) |
| Cocamine Oxide | 1.2 g | Lonza (Barlox 12) |
| Poloxmer 124 | 1.0 g | BASF (Poloxmer 124) |
| Sodium Coco PG-Dimonium Chloride Phosphate | 1.8 g | Uniqema (Arlasilk Phospholipid CDN |
| RBG-9M | 0.2 g | Rita (Rita PEO-2) |
| Lactic acid | q.s. to pH 5.25 | Purac (Purac HiPure USP 90%) |

Table Two:

| <u>Organism</u> | <u>ATCC Number</u> | <u>Exposure Time</u> | <u>Log Reduction</u> | <u>Percent Reduction</u> |
|---|---|---|---|---|
| *Acinetobacter baumannii* | 19606 | 15 sec | 6.6484 | 99.9999% |
| | | 30 sec | 6.6484 | 99.9999% |

(continued)

| Organism | ATCC Number | Exposure Time | Log Reduction | Percent Reduction |
|---|---|---|---|---|
| *Aspergillus flavus* | 9643 | 15 sec | 0.0000 | 0.0000% |
| | | 30 sec | 0.0000 | 0.0000% |
| *Aspergillus niger* | 9642 | 15 sec | 0.0808 | 16.9811% |
| | | 30 sec | 0.1010 | 20.7574% |
| *Bacillus megaterium* | 14581 | 15 sec | 6.0737 | 99.9999% |
| | | 30 sec | 6.0737 | 99.9999% |
| *Bacteroides fragilis* | 29762 | 15 sec | 7.8325 | 99.9999% |
| | | 30 sec | 7.8325 | 99.9999% |
| *Burkholderia cepacia* | 25461 | 15 sec | 1.3596 | 95.6311% |
| | | 30 sec | 2.0990 | 99.2039% |
| *Campylobacter jejuni* | 29428 | 15 sec | 3.2380 | 99.9422% |
| | | 30 sec | 3.2380 | 99.9422% |
| *Candida albicans* | 14053 | 15 sec | 0.0494 | 10.7527% |
| | | 30 sec | 0.0000 | 0.0000% |
| *Candida tropicalis* | 13803 | 15 sec | 1.6859 | 97.9391% |
| | | 30 sec | 3.3166 | 99.9518% |
| *Citrobacter freundii* | 8090 | 15 sec | 6.7363 | 99.9999% |
| | | 30 sec | 6.7364 | 99.9999% |
| *Clostridium difficile* | 9689 | 15 sec | 6.1875 | 99.9999% |
| | | 30 sec | 6.1875 | 99.9999% |
| *Clostridium perfringens* | 13124 | 15 sec | 6.7482 | 99.9999% |
| | | 30 sec | 6.7482 | 99.9999% |
| *Corynebacterium diphtheriae* | 11913 | 15 sec | 5.4314 | 99.9996% |
| | | 30 sec | 5.4314 | 99.9996% |
| *Enterococcus faecalis* | 51575 | 15 sec | 4.8838 | 99.9987% |
| | | 30 sec | 6.4023 | 99.9999% |
| *Enterococcus faecalis* | 29212 | 15 sec | 6.1089 | 99.9999% |
| | | 30 sec | 6.1089 | 99.9999% |
| *Enterococcus faecium* | 51559 | 15 sec | 3.1022 | 99.9210% |
| | | 30 sec | 4.8893 | 99.9987% |
| *Epidermophyton floccosum* | 52066 | 15 sec | 1.7386 | 98.1746% |
| | | 30 sec | 2.4983 | 99.6825% |
| *Escherichia coli* | 11229 | 15 sec | 5.9823 | 99.9999% |
| | | 30 sec | 5.9823 | 99.9999% |
| *Escherichia coli* | 25922 | 15 sec | 5.8513 | 99.9999% |
| | | 30 sec | 5.8513 | 99.9999% |
| *Escherichia coli* | 43888 | 15 sec | 5.9590 | 99.9999% |
| | | 30 sec | 5.9590 | 99.9999% |

(continued)

| Organism | ATCC Number | Exposure Time | Log Reduction | Percent Reduction |
|---|---|---|---|---|
| *Haemophilus influenzae* | 33930 | 15 sec | 5.4116 | 99.9996% |
| | | 30 sec | 5.4116 | 99.9996% |
| *Klebisella pheumoniae* | 11296 | 15 sec | 6.2201 | 99.9999% |
| | | 30 sec | 6.2201 | 99.9999% |
| *Klebsiella pneomoniae* | 13883 | 15 sec | 6.2019 | 99.9999% |
| | | 30 sec | 6.2019 | 99.9999% |
| *Lactobacillus plantarum* | 14917 | 15 sec | 5.9445 | 99.9999% |
| | | 30 sec | 5.9445 | 99.9999% |
| *Listeria monocytogenes* | 7644 | 15 sec | 5.3174 | 99.9995% |
| | | 30 sec | 5.2009 | 99.9994% |
| *Penicillum citrinum* | 9849 | 15 sec | 0.3052 | 50.4673% |
| | | 30 sec | 0.4612 | 65.4206% |
| *Proteus mirabilis* | 7002 | 15 sec | 2.9755 | 99.8942% |
| | | 30 sec | 3.8663 | 99.9864% |
| *Proteus vulgaris* | 13315 | 15 sec | 6.0607 | 99.9999% |
| | | 30 sec | 6.0607 | 99.9999% |
| *Pseudomonas aeruginosa* | 15442 | 15 sec | 5.7709 | 99.9998% |
| | | 30 sec | 5.7709 | 99.9998% |
| *Pseudomonas aeruginosa* | 27853 | 15 sec | 6.5911 | 99.9999% |
| | | 30 sec | 6.5911 | 99.9999% |
| *Salmonella choleraesuis* | 10708 | 15 sec | 6.1614 | 99.9999% |
| | | 30 sec | 6.1614 | 99.9999% |
| *Salmonella choleraesius* | 13076 | 15 sec | 5.8751 | 99.9999% |
| | | 30 sec | 5.8751 | 99.9999% |
| *Salmonella choleraesuis* | 14028 | 15 sec | 5.9845 | 99.9999% |
| | | 30 sec | 5.9845 | 99.9999% |
| *Serratia marcesens* | 14756 | 15 sec | 2.5790 | 99.7363% |
| | | 30 sec | 4.2691 | 99.9946% |
| *Shigella dysenteriae* | 13313 | 15 sec | 5.8129 | 99.9998% |
| | | 30 sec | 5.8129 | 99.9998% |
| *Shigella sonnei* | 11060 | 15 sec | 6.0569 | 99.9999% |
| | | 30 sec | 6.0569 | 99.9999% |
| *Staphylococcus aureus* | 6538 | 15 sec | 0.9944 | 89.8708% |
| | | 30 sec | 1.5308 | 97.0543% |
| *Staphylococcus aureus* | 29213 | 15 sec | 2.2488 | 99.4360% |
| | | 30 sec | 3.3641 | 99.9568% |
| *Staphyloccus aureus* | 33591 | 15 sec | 1.6617 | 97.8211% |
| | | 30 sec | 2.6877 | 99.7947% |

(continued)

| Organism | ATCC Number | Exposure Time | Log Reduction | Percent Reduction |
|---|---|---|---|---|
| *Staphylococcus epidermidis* | 12228 | 15 sec | 4.3365 | 99.9954% |
| | | 30 sec | 4.3365 | 99.9954% |
| *Staphylococcus haemolyticus* | 43253 | 15 sec | 4.5250 | 99.9970% |
| | | 30 sec | 4.5250 | 99.9970% |
| *Staphyloccus hominis* | 27845 | 15 sec | 3.9542 | 99.9889% |
| | | 30 sec | 3.9542 | 99.9889% |
| *Staphylococcus saprophyticus* | 49453 | 15 sec | 4.6580 | 99.9978% |
| | | 30 sec | 4.6580 | 99.9978% |
| *Streptococcus pneumoniae* | 33400 | 15 sec | 4.7520 | 99.9982% |
| | | 30 sec | 4.7520 | 99.9982% |
| *Streptococcus pyogenes* | 19615 | 15 sec | 6.6721 | 99.9999% |
| | | 30 sec | 6.6721 | 99.9999% |
| *Trichophyton mentagrophytes* | 9533 | 15 sec | 0.1416 | 27.8302% |
| | | 30 sec | 0.1960 | 36.3208% |

The hand wash exhibited broad, fast acting antimicrobial activity against Gram-positive and Gram-negative bacteria. There was a limited degree of efficacy against molds, but the log kill was sufficient to not impede the use of this hand wash as a sanitizer. Of the 46 organisms tested the hand wash had greater than 5.0 log reduction on 26 of the organisms; 7 organisms had greater than 4.0 log reduction; 5 organisms had greater than 3.0 log reduction; 3 organisms had greater than 2.0 log reduction; 1 organism had greater than 1.0 log reduction; and the remaining four had below one log reduction. The four organisms that had less than one log reduction were *Aspergillus niger, Trichophyton mentagrophytes, Penicillum citrinum* and *Aspergillus flavus,* all of which are fungus colonies.

Example Three:

As disclosed, hand washes made in accordance with the particularly preferred embodiments of this invention have a reduced level of solids. To show this, the solids content of the hand wash formulation of Example Two, above, was compared with competitive antimicrobial hand washes. Competitive antimicrobial hand washes were tested for solids content by placing a weighed sample into a 50°C oven for 48 hours to ensure all the volatile components were removed. After the 48 hours the samples were removed from the oven and allowed to cool to room temperature. Then the samples were weighed a second time and the percent solids calculated from the two values.

$$\text{Percent solids} = 1 - \frac{(\text{Mass}_{Start} - \text{Mass}_{Left})}{\text{Mass}_{Start}}$$

Table Three:

| Hand Wash | Percent Solids (%w/w) |
|---|---|
| Dial Complete - Foaming Hand Wash | 27.96 |
| Dial Complete - HCPHW | 31.88 |
| Flora Free | 15.50 |
| Acute-Kare | 12.86 |
| Foam Care | 12.68 |
| Bacti-Stat | 16.27 |

(continued)

| Table Three: | |
|---|---|
| Hand Wash | Percent Solids (%w/w) |
| Medi-Scrub | 17.74 |
| Bacti-Foam | 15.70 |
| Endure 250 | 18.74 |
| Keystone | 18.62 |
| | |
| Average: | 18.95 |

The total solids amount of the hand wash of Example Two was 8.4 weight percent. Compare this value to the average value from the competitive hand washes, 18.95 weight percent. There is over a fifty percent reduction in the total solids content of the hand wash.

Example Four:

Because of the reduced amount of solids in the present hand washes, the irritancy of the hand wash is minimized. As seen in Example Three, the solids of a hand wash described herein has solids content that is nearly 50% of the average for competitive antimicrobial hand washes. This reduction in solids shows a positive effect on reducing the irritancy of the hand wash. In this the hand wash of Example Two was diluted in deionized water to one percent of the original volume. The sample was then applied to a patch. This patch, after being soaked in the solution, was placed on a test subject's skin for a set amount of time. After the patch sat on the skin for the required time, moisture measurements and redness values were assigned. Also similarly tested were both positive, sodium lauryl sulfate, and negative, Johnson and Johnson Baby Oil, controls. These two samples were diluted to 0.2 percent of the original volume into deionized water.

Table Four:

| Sample | Irritancy Score | Classification |
|---|---|---|
| Prototype | 24.5 | Mild |
| Johnson and Johnson Baby Oil (Neg Control) | 39.5 | Mild |
| Sodium Lauryl Sulfate (Pos Control) | 14918.5 | Experimental Cumulative Irritant |

The scale or classification is as follows:

| Category | Description | Range |
|---|---|---|
| I | Mild | 0-155 |
| II | Probably Mild | 156-620 |
| III | Possibly Mild | 621-1399 |
| IV | Experimental Cumulative Irritant | 1400-1807 |
| V | Experimental Primary Irritant | 1808-1953 |

Per Table Four, the hand wash of this invention has a very low irritancy score, lower in fact than the negative control. This low irritation comes from the reduction of solids in the hand wash, because some of the compounds found in the hand wash are appreciated as irritants, primarily the surfactants. Their reduction ensures a non-irritating hand wash, which fits with one of the criteria for use as a repeated hand wash.

Example Five:

One of the desires of end users for hand washes is copious amounts of foam during the hand wash experience. One way to test the amount and type of foam created using a hand wash is to dilute the hand wash sample into water and then shake the sample to produce foam. The process used here was a 100 fold dilution of 1 mL of the hand wash of Example Two into deionized water. This solution was mixed gently to prevent foaming and then added to a 500 mL

graduated cylinder. The cylinder was stoppered and the sample was inverted ten times. The sample was then set on a flat surface, the top was removed, and the sample sat for 5 minutes. Once the inversions were done, the height of the foam was recorded and the volume of liquid was subtracted from this to yield the height of the foam. After 5 minutes the foam height was measured again and the volume of liquid was subtracted from this.

The first measurement of the foam height gives an indication of the flash foaming ability of the hand wash. This means the hand wash's ability to produce quick amounts of foam requiring little mechanical force to generate it. The second measurement is a relative measure of the stability of the foam. While washing, the foam deteriorates, but the longer the foam lasts in this test, the more likely it will last during a hand wash.

Table Five:

| Hand Wash Name | Flash | Stable |
|---|---|---|
| Keystone | 210 | 160 |
| Dial Complete HCPHW | 130 | 105 |
| Endure | 160 | 130 |
| Bacti-Foam | 150 | 135 |
| Flora Free | 370 | 250 |
| Dial Complete Foaming | 250 | 145 |
| Bacti-Stat | 180 | 170 |
| Foam Care | 290 | 270 |
| Acute-Kare | 85 | 75 |
| Medi-Scrub | 220 | 200 |
| Hibiclean | 240 | 220 |
| Example Two Hand Wash | 190 | 145 |

The product on the bottom of the prototype disclosed herein. Although the hand wash of Example Two has much less solids than competitive counterparts, the foam generated was near the average of all the other samples and well within one standard deviation of this norm.

In light of the forgoing, it should be evident that the present invention provides improvements in hand wash formulations and their method of manufacture.

The following claims will establish the scope of this invention.

## Claims

**1.** An aqueous antimicrobial hand wash comprising:

a phenol-based active ingredient selected from 2,3,4'-trichloro-2'-hydroxydiphenylether, 2,2'-dihydroxy-5,5'-dibromodiphenyl ether, p-chloro-m-xylenol, ortho-phenylphenol, and mixtures thereof;
an amphoteric surfactant selected from the group consisting of disodium capryloamphodiacetate and cocaminopropionic acid; and
a quaternary ammonium compound selected from the group consisting of polyethylene glycol (PEG) derivatives of a quaternary ammonium salt of a fatty amine, polypropylene glycol (PPG) derivatives of a quaternary ammonium salt of a fatty amine, and mixtures thereof, wherein said quaternary ammonium compound dissolves at least a portion of said phenol-based active ingredient.

**2.** The antimicrobial hand wash of claim 1, wherein said quaternary ammonium compound is a PEG derivative selected from the group consisting of PEG-5 oleammonium methosulfate, PEG-2 oleammonium chloride, PEG-15 oleammonium chloride and mixtures thereof.

**3.** The hand wash of claim 1, wherein the hand wash is comprised of from 0.01 to 10 wt% of said phenol-based active ingredient.

**4.** The hand wash of claim 4, wherein the hand wash is comprised of from 0.05 to 25 wt% of said amphoteric surfactant.

**5.** The hand wash of claim 5, wherein the hand wash is comprised of from 0.01 to 10 wt% of said quaternary ammonium compound.

6. A method for producing an aqueous antimicrobial hand wash comprising the steps of:

creating an active ingredient premix comprised of a phenol-based active ingredient selected from 2,3,4'-trichloro-2'-hydroxydiphenylether, 2,2'-dihydroxy-5,5'-dibromodiphenyl ether, p-chloro-m-xylenol, ortho-phenylphenol, and mixtures thereof and a quaternary ammonium compound selected from polyethylene glycol (PEG) derivatives of a quaternary ammonium salt of a fatty amine, polypropylene glycol (PPG) derivatives of a quaternary ammonium salt of a fatty amine, and mixtures thereof, wherein the quaternary ammonium compound dissolves at least a portion of the phenol-based active ingredient;
creating a second premix of water and a supplemental surfactant package including at least one amphoteric surfactant selected from the group consisting of disodium capryloamphodiactate and cocaminopropionic acid; and
adding the active ingredient premix to the second premix.

## Patentansprüche

1. Wässrige antimikrobielle Handseife, umfassend:

einen Wirkstoff auf Phenolbasis, ausgewählt aus 2,3,4'-Trichlor-2'-hydroxydiphenylether, 2,2'-Dihydroxy-5,5'-dibromdiphenylether, p-Chlor-m-xylenol, Orthophenylphenol sowie deren Mischungen;
ein amphoteres Tensid, ausgewählt aus der Gruppe bestehend aus Dinatriumcapryloamphodiacetat und Cocaminpropansäure; und
eine quartäre Ammoniumverbindung, ausgewählt aus der Gruppe bestehend aus Polyethylenglycolderivaten (PEG) eines quartären Ammoniumsalzes eines Fettamins, Polypropylenglycolderivaten (PPG) eines quartären Ammoniumsalzes eines Fettamins sowie deren Mischungen, wobei die genannte quartäre Ammoniumverbindung wenigstens einen Teil des genannten Wirkstoffs auf Phenolbasis auflöst.

2. Antimikrobielle Handseife nach Anspruch 1, wobei es sich bei der genannten quartären Ammoniumverbindung um ein PEG-Derivat handelt, das ausgewählt ist aus der Gruppe bestehend aus PEG-5-oleammoniummethosulfat, PEG-2-oleammoniumchlorid, PEG-15-oleammoniumchlorid sowie deren Mischungen.

3. Handseife nach Anspruch 1, wobei die Handseife von 0,01 bis 10 Gewichts-% des genannten Wirkstoffs auf Phenolbasis umfasst.

4. Handseife nach Anspruch 4, wobei die Handseife von 0,05 bis 25 Gewichts-% des genannten amphoteren Tensids umfasst.

5. Handseife nach Anspruch 5, wobei die Handseife von 0,01 bis 10 Gewichts-% der genannten quartären Ammoniumverbindung umfasst.

6. Verfahren zur Herstellung einer wässrigen antimikrobiellen Handseife, umfassend die nachfolgenden Schritte:

Herstellen einer Wirkstoffvormischung, umfassend einen Wirkstoff auf Phenolbasis, ausgewählt aus 2,3,4'-Trichlor-2'-hydroxydiphenylether, 2,2'-Dihydroxy-5,5'-dibromodiphenylether, p-Chlor-m-xylenol, Orthophenylphenol sowie deren Mischungen und eine quartäre Ammoniumverbindung, ausgewählt aus Polyethylenglycolderivaten (PEG) eines quartären Ammoniumsalzes eines Fettamins, Polypropylenglycolderivaten (PPG) eines quartären Ammoniumsalzes eines Fettamins sowie deren Mischungen, wobei die quartäre Ammoniumverbindung wenigstens einen Teil des genannten Wirkstoffs auf Phenolbasis auflöst;
Herstellen einer zweiten Vormischung aus Wasser und einem ergänzenden Tensidpaket, einschließlich wenigstens einem amphoteren Tensid, das ausgewählt ist aus der Gruppe bestehend aus Dinatriumcaprylamphodiactat und Cocaminopropansäure; und
Hinzufügen der Wirkstoffvormischung zur zweiten Vormischung.

## Revendications

1. Produit de lavage antimicrobien aqueux pour les mains comprenant :

un principe actif à base de phénol sélectionné parmi le 2,3,4'-trichloro-2'-hydroxydiphényléther, le 2,2'-dihydroxy-5,5'-dibromodiphényl éther, le p-chloro-m-xylénol, l'ortho-phénylphénol et leurs mélanges ;
un tensioactif amphotère sélectionné dans le groupe constitué de capryloamphodiacétate de disodium et d'acide aminopropionique dérivé de coco ; et
un composé d'ammonium quaternaire sélectionné dans le groupe constitué de dérivés de polyéthylène glycol (PEG) d'un sel d'ammonium quaternaire d'une amine grasse, de dérivés de polypropylène glycol (PPG) d'un sel d'ammonium quaternaire d'une amine grasse, et leurs mélanges, dans lequel ledit composé d'ammonium quaternaire dissout au moins une partie dudit principe actif à base de phénol.

**2.** Produit de lavage antimicrobien pour les mains selon la revendication 1, dans lequel ledit composé d'ammonium quaternaire est un dérivé de PEG sélectionné dans le groupe constitué de méthosulfate de PEG-5 oléammonium, de chlorure de PEG-2 oléammonium, de chlorure de PEG-15 oléammonium et de leurs mélanges.

**3.** Produit de lavage pour les mains selon la revendication 1, dans lequel le produit de lavage pour les mains est composé de 0,01 à 10 % en poids dudit principe actif à base de phénol.

**4.** Produit de lavage pour les mains selon la revendication 4, dans lequel le produit de lavage pour les mains est composé de 0,05 à 25 % en poids dudit tensioactif amphotère.

**5.** Produit de lavage pour les mains selon la revendication 5, dans lequel le produit de lavage pour les mains est composé de 0,01 à 10 % en poids dudit composé d'ammonium quaternaire.

**6.** Procédé de production d'un produit de lavage antimicrobien aqueux pour les mains comprenant les étapes suivantes :

créer un pré-mélange de principe actif composé d'un principe actif à base de phénol sélectionné parmi le 2,3,4'-trichloro-2'-hydroxydiphényléther, le 2,2'-dihydroxy-5,5'-dibromodiphényl éther, le p-chloro-m-xylénol, l'ortho-phénylphénol et leurs mélanges et d'un composé d'ammonium quaternaire sélectionné parmi des dérivés de polyéthylène glycol (PEG) d'un sel d'ammonium quaternaire d'une amine grasse, des dérivés de polypropylène glycol (PPG) d'un sel d'ammonium quaternaire d'une amine grasse et leurs mélanges, dans lequel le composé d'ammonium quaternaire dissout au moins une partie du principe actif à base de phénol;
créer un deuxième pré-mélange d'eau et un ensemble supplémentaire de tensioactifs comprenant au moins un tensioactif amphotère sélectionné dans le groupe constitué de capryloamphodiacétate de disodium et d'acide aminopropionique dérivé de coco ; et
ajouter le pré-mélange de principe actif dans le deuxième pré-mélange.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0141567 A